# EUROPEAN PATENT APPLICATION

(11) **EP 0 974 593 A1**
(43) Date of publication of application: **26.01.2000**
(21) Application number: 98305592.2
(22) Date of filing: 14.07.1998
(51) Int. Cl.: C07D 491/20, A61K 31/435

(54) **Artemisinin derivatives as antiparasitic agents**

(71) Applicant: The Hong Kong University of Science & Technology, Kowloon (HK)
(72) Inventor: Haynes, Richard Kingston, Kowloon (HK); Lam, Wai-Lun, Kowloon (HK)
(74) Representative: Wallace, Sheila Jane

(57) **Abstract**

This invention relates to the use of certain 11-aza derivatives of artemisinin in the treatment and/or prophylaxis of diseases caused by infection with a parasite, certain novel 11-aza derivatives of artemisinin, processes for their preparation and pharmaceutical compositions containing such 11-aza derivatives. The compounds are particularly effective in the treatment of malaria, neosporosis and coccidiosis.

## Description

This invention relates to the use of certain 11-aza derivatives of artemisinin in the treatment and/or prophylaxis of diseases caused by infection with a parasite, pharmaceutical compositions containing such 11-aza derivatives, certain novel 11-aza derivatives of artemisinin and processes for their preparation.

Malaria is the most important human parasitic disease in the world today. Approximately 270 million people throughout the world are infected with malaria, with about 2 million dying each year. The ability of parasites to produce a complex survival mechanism by expressing variant antigens on the surface of infected erythrocytes makes it possible for the parasites to escape from the destructive action of the host immune response against these antigens. In addition, the increasing rate of malaria infection is due to the spread of chloroquine-resistant strains of Plasmodium falciparum and the other multi-drug resistant strains.

In the field of animal health, parasitic diseases are a major problem, especially those diseases which are functionally related to malaria. For instance, neosporosis is a term used to describe diseases caused by parasites of the species Neospora, especially Neospora caninum, in animals. Neospora infections are known to occur in dogs, cattle, sheep, goats and horses.

The final host for Neospora spp., including Neospora caninum, is unknown and, in addition, the complete cycle of development of the parasite is not understood. The asexual phases of reproduction, known as schizogony, and the behaviour of the unicellular tachyzoite/bradyzoite stage have been clarified, however. Tachyzoites are infectious unicellular parasite stages of about 3-7 x 1-5 mm in size formed after intracellular reproduction termed endodyogeny. Reproduction via tachyzoites takes place preferentially in organelles such as muscle or nerve cells. Pathological symptoms invoked after an infection are associated mainly in those tissues. Some five to six weeks after natural infection in a dog, symptoms of the disease are hypersensitivity caused by inflammation of neuronal cells and increasing tendency to hyperextension of the hind legs. Histopathological lesions are apparent in the nervous system, preferentially in the brain and spinal cord. Extensive non-suppurative inflammations, glial excrescences and perivascular infiltrations of mononuclear cells (macrophages, lymphocytes, plasma cells) dominate, and are also partly apparent in eosinophils and neutrophils. In the muscular system, macroscopically observable necroses and degenerative changes appear. Apart from the more or less strongly developed atrophy, long pale longitudinal stripes are evident.

In California and Australia, Neospora caninum infections appear to be the main cause for abortion in cattle. Symptoms of the disease in cattle are similar to those in the dog. Ataxia is apparent, joint reflexes are weakened and pareses at the hind legs, partly in all four legs, can be observed. The histological picture is similar to that of the dog; mainly non-suppurative meningitis and myelitis.

Data on in vivo activity of compounds suitable against neosporosis are rare because adequate in vivo test systems still have to be developed. Sulfadiazin (administered via drinking water) is effective in experimentally infected mice, only if the treatment was prophylactic, that is, the treatment was started before infection. In dogs, treatment with sulfadiazin and clindamycin is only successful if it is started early, that is, at the appearance of first clinical symptoms as a result of neuronal inflammation.

Coccidiosis, an infection of the small intestine, is relatively rarely diagnosed in humans, where it is caused by Isospora belli. However, humans are also the final host of at least two cyst-forming coccidial species (Sarcocystis suihominis and S. bovihominis). Consumption of raw or inadequately cooked pork or beef containing such cysts can lead to severe diarrhoea, the cause of which is probably seldom diagnosed correctly. Coccidia (phylum Apicomplexa, suborder Eimeriina) are one of the most successful groups of parasitic protozoans, having conquered virtually every class of Metazoa. The ones that are of particular importance for man are the 60-100 species which parasitise domestic animals and which in some instances can cause very severe losses, especially in poultry, although also in lambs, calves, piglets, rabbits and other animals (see Table A).

**Table A:**

| Causatives of intestinal coccidiosis in domestic animals | | |
|---|---|---|
| Animal | number of Eimeria and/or Isospora species*) | most pathogenic and/or very common species (E=Eimeria, I=Isospora) |
| chicken (Gallus gallus) | 7 | E.tenella, E.necatrix, E.maxima, E.acervulina |
| turkey (Meleargidis gallopavo) | 7 | E.meleagrimitis, E.adenoides |
| goose (Anser anser) | 6 | E.anseris, E.truncata, E.nocens, E. kotlani |
| duck (Anas platyhynehus) | 3 | Tyzzeria perniciosa, E.anatis |
| pigeon (Columba livia) | 2 | E.columbarum, E.labbeanea |
| rabbit (Oryctolagus cuniculus) | 11(12) | E.intestinalis, E.flavescens, E.stiedai, E.magna, E.perforans |
| sheep (Ovis arius) | 11(16) | E.ovinoidalis, E.ashataE.o vina |
| goat (Capra hircus) | 12(15) | E.ninakohlyakimovae, E.arloingi |
| cattle (Bos taurus) | 12(15) | E.zuernii, E.bovis, E.auburnensis |
| pig (Sus scofra) | 7(14) | I.suis, E.debliecki, E.scabra |
| dog (Canis familiaris) | 5 | I.canis, I.(Cystisospora) burrowsi |
| cat (Felis catus) | 2+6 | I.felis, I.rivolta as final host: Sarcocystis bovifelis, S.ovifelis, S.fusiformis, S.muris, S.cuniculi, Toxoplasma gondii |

| | | |
|---|---|---|
| *) regarding to Pellerdy (1974), Eckert et al, (1995b, Levine and Ivens (1970) and Mehlhorn 1988) | | |

Most of the pathogenic species are strictly host-specific. They have a complex life cycle with two asexual reproduction phases (schizogony or merogony, and sporogony) and a sexual development phase (gametogony). In view of the major importance of coccidiosis, numerous reviews are available, for instance, by Davies et al. (1963), Hammond and Long (1973), Long (1982, 1990), and Pellerdy (1974). The economically important species sometimes differ very considerably in their sensitivity to medicinal active ingredients. The sensitivity of the different developmental stages to medicinal agents also varies enormously.

As far as the use of drugs is concerned, prophylaxis is the main approach in poultry, in which symptoms do not appear until the phase of increased morbidity, and therapy is the principal strategy in mammals (McDougald 1982). Polyether antibiotics and sulfonamides, among other drugs, are currently used for such treatment and prophylaxis. However, drug-resistant strains of Eimeria have emerged and drug-resistance is now a serious problem. New drugs are therefore urgently required. Given the multiplicity of pathogens and hosts, there is no "ideal model" for identifying and testing anticoccidial agents. For example, most of the many substances used for preventing coccidiosis in poultry are insufficiently effective or even completely ineffective against mammalian coccidia (Haberkorn and Mundt; 1989; Haberkorn 1996). Numerous works and sets of instructions have been published on testing of active ingredients in animals for anticoccidial efficacy, for immunisation, etc. One particularly important and comprehensive example is the survey of current methods published by Eckert et al. (1995a).

The compound artemisinin, also known as qinghaosu (1), is a tetracyclic 1,2,4-trioxane occurring in Artemisia annua. Artemisinin and its derivatives dihydroartemisinin (2), artemether (3) and sodium artesunate (4) have been used for the treatment of malaria.

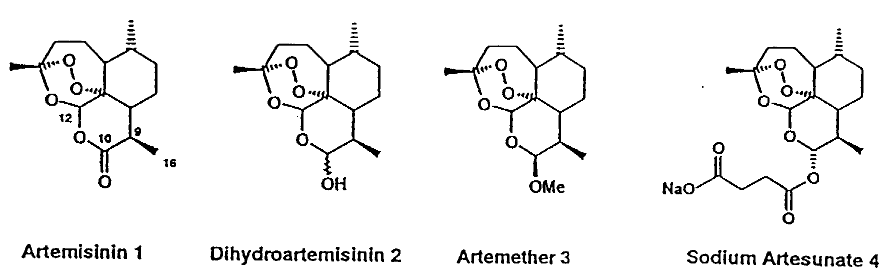

Different modes of action have been proposed by various groups to account for the action of artemisinin and its derivatives in treating malaria (Posner et al., *J.Am.Chem.Soc.**1996**,118,3537*;Posner at al., *J.Am.Chem.Soc.**1995**,117,5885*;Posner at al., J. *Med. Chem.**1995**, 38, 2273).* However, irrespective of actual mode of action, all current derivatives suffer from poor oral bioavailability and poor stability (Meshnick et al., *Parasitology Today **1996**,12,79),* especially the 'first generation' ethers and esters artemether and sodium artesunate obtained from dihydroartemisinin. Extensive chemical studies carried out on artemisinin and derivatives indicate that a cause of instability is the facile opening of the trioxane moiety in artemisinin itself, or in the metabolite common to all currently used derivatives artemether, arteether and artesunate, namely dihydroartemisinin. Ring opening will provide the free hydroperoxide, which is susceptible to reduction. Removal of this group ensures destruction of drug activity with the reduction products being transformed into desoxo metabolites. In order to render ring-opening less facile, the oxygen atom at C-10 can be either removed to provide 10-deoxydihydroartemisinin, or replaced by other groups, and this has provided the basis for the so-called 'second generation' compounds which are generally 10-deoxy artemisinin derivatives. In addition, derivatives of artemisinin have also been prepared with a variety of substituents at C-9.

Artemisinin derivatives are also known in which the oxygen atom at the 11-position has been replaced by a nitrogen atom to produce ll-azaartemisinin and N-substituted derivatives thereof. For instance, Torok and Ziffer *(J.Med.Chem.,1995,38,5045-5050)* synthesised ll-azaartemisinin and derivatives thereof in which the 11-nitrogen atom is substituted by a methyl, isobutyl, allyl, formylmethyl, benzyl, pyrid-2-ylmethyl, 2-thiophenemethyl or furfuryl group. These compounds were tested for in vitro activity against a chloroquine-resistant strain of Plasmodium falciparum and all compounds except the N-benzyl derivatives exhibited some activity. Mekonnen and Ziffer *(Tetrahedron Letters,**1997**,38(5),731-734)* also synthesised 11-azaartemisinin and its N-allyl and N-formylmethyl derivatives and described the conversion of 11-azaartemisinin by a Michael addition reaction to derivatives in which the 11-nitrogen atom is substituted by a cyanoethyl, ethoxycarbonylethyl, methylcarbonylethyl, phenylsulphinylethyl, phenylsulphonylethyl or phenylsulphonatoethyl group. However, these derivatives were not tested for biological activity.

Whilst the current artemisinin derivatives are successful, there are problems associated with stability, bioavailability and potential neurotoxicity. There is also a need for artemisinin derivatives which exhibit a broad spectrum of activity against a variety of parasites.

It has now been discovered that ll-azaartemisinin and certain N-substituted derivatives thereof are effective in the treatment of diseases caused by infection with a parasite. These compounds are particularly effective in the treatment of malaria, neosporosis and coccidiosis, especially forms of these diseases caused by infection with a parasite of the genera Plasmodium, Neospora or Eimeria, particularly Plasmodium falciparum, Neospora caninum and Eimeria tenella. According to the present invention there is therefore provided a compound of the general 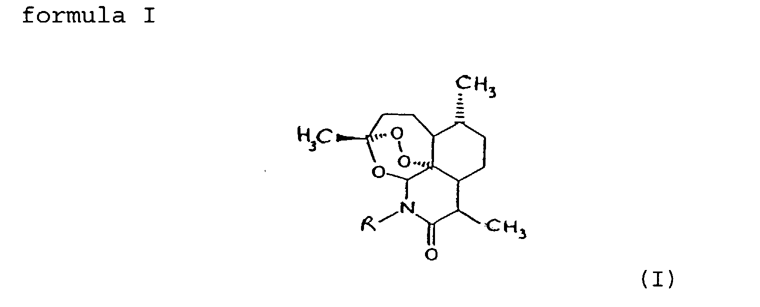 in which
R represents a hydrogen atom or an optionally substituted alkyl, alkenyl, alkynyl, aralkyl or heterocyclylalkyl group;
for use in the treatment and/or prophylaxis of a disease caused by infection with a parasite other than an organism of the genus Plasmodium

Any alkyl, alkenyl or alkynyl group, unless otherwise specified, may be linear or branched and may contain up to 12, preferably up to 6, and especially up to 4 carbon atoms. Preferred alkyl groups are methyl, ethyl, propyl and butyl. It is preferred that any alkenyl or alkynyl group is not an alk-1-enyl or alk-1-ynyl group. In other words, there should preferably be at least one methylene group -CH₂- or similar sp³-hybridised centre between a carbon atom forming part of the double or triple C-C bond and the ring nitrogen atom to which the group is attached. Preferred alkenyl and alkynyl groups are propenyl, butenyl, propynyl and butynyl groups. When an alkyl moiety forms part of another group, for example the alkyl moiety of an aralkyl or heterocyclylalkyl group, it is preferred that it contains up to 6, especially up to 4, carbon atoms. Preferred alkyl moieties are methyl and ethyl.

An aralkyl group may be any alkyl group substituted by an aryl group. The alkyl moiety may be as defined above and the aryl moiety may be any aromatic hydrocarbon group and may contain from 6 to 24, preferably 6 to 18, more preferably 6 to 16, and especially 6 to 14 carbon atoms. Preferred aryl moieties include phenyl, naphthyl, anthryl, phenanthryl and pyryl, especially phenyl and naphthyl, moieties. A preferred aralkyl group contains from 7 to 30, particularly 7 to 24 and especially 7 to 18 carbon atoms, particularly preferred aralkyl groups being benzyl, naphthylmethyl, anthrylmethyl, phenanthrylmethyl and pynylmethyl groups. An especially preferred aralkyl group is a benzyl group.

A heterocyclylalkyl group may be any alkyl group substituted by a heterocyclic group. In this context, a heterocyclic group may be any monocyclic or polycyclic ring system which contains at least one heteroatom and may be unsaturated or partially or fully saturated. The term "heterocyclic" thus includes aromatic heterocyclic groups termed "heteroaryl" groups as well as non-aromatic heterocyclic groups. A heteroaryl group may be any aromatic monocyclic or polycyclic ring system which contains at least one heteroatom. Preferably, a heteroaryl group is a 5- to 18- membered, especially a 5- to 14-membered, aromatic ring system containing at least one heteroatom selected from oxygen, sulphur and nitrogen atoms. Preferred heteroaryl groups include pyridyl, pyrylium, thiopyrylium, pyrrolyl, furyl, thienyl, indolinyl, isoindolinyl, indolizinyl, imidazolyl, pyridonyl, pyronyl, pyrimidinyl, pyrazinyl, oxazolyl, thiazolyl, purinyl, quinolinyl, isoquinolinyl, quinoxalinyl, pyridazinyl, benzofuranyl and acridinyl groups. Preferably, a heterocyclic group is a 3- to 18- membered, particularly a 3- to 14-membered and especially a 5- to 10-membered, ring system containing at least one heteroatom selected from oxygen, sulphur and nitrogen atoms. Preferred heterocyclic groups include the specific heteroaryl groups named above as well as pyranyl, piperidinyl, pyrrolidinyl, dioxanyl, piperazinyl, morpholinyl, benzoxazolyl, indolinyl, tetrahydroisoquinolinyl and tetrahydrofuranyl groups. Preferred heterocyclylalkyl groups therefore include imidazolylmethyl, pyrrolylmethyl, pyridylmethyl, pyrimidinylmethyl, pyrazolylmethyl, furylmethyl (especially furfuryl), pyranylmethyl, thienylmethyl (especially thenyl), oxazolylmethyl, thiazolylmethyl, quinolinylmethyl, indolylmethyl and benzofuranylmethyl.

When any of the foregoing substituents are designated as being optionally substituted, the substituent groups which are optionally present may be any one or more of those customarily employed in the development of pharmaceutical compounds and/or the modification of such compounds to influence their structure/activity, stability, bioavailability or other property. Specific examples of such substituents include, for example, halogen atoms, nitro, cyano, hydroxyl, cycloalkyl, alkyl, haloalkyl, alkoxy, haloalkoxy, amino, alkylamino, dialkylamino, formyl, alkoxycarbonyl, carboxyl, alkanoyl, alkylthio, alkylsulphinyl, alkylsulphonyl, alkylsulphonato, arylsulphinyl, arylsulphonyl, arylsulphonato, carbamoyl and alkylamido groups. When any of the foregoing substituents represents or contains an alkyl substituent group, this may be linear or branched and may contain up to 12, preferably up to 6, and especially up to 4, carbon atoms. A cycloalkyl group may contain from 3 to 8, preferably from 3 to 6, carbon atoms. An aryl group or moiety may contain from 6 to 10 carbon atoms, phenyl groups being especially prefered. A halogen atom may be a fluorine, chlorine, bromine or iodine atom and any group which contains a halo moiety, such as a haloalkyl group, may thus contain any one or more of these halogen atoms.

It is preferred that R represents a hydrogen atom or a C₁₋₆ alkyl, C₂₋₆ alkenyl, C₂₋₆alkynyl, C₇₋₁₆ aralkyl or 5- to 10-membered-heterocyclic-C₁₋₆ alkyl group, each group being optionally substituted by one or more substituents selected from the group consisting of halogen atoms, cyano, nitro, C₁₋₄ haloalkyl, formyl, C₁₋₆ alkoxycarbonyl, C₁₋₆ alkanoyl, phenylsulphinyl, phenylsulphonyl and phenylsulphonato groups.

It is also preferred that R represents a hydrogen atom or a C₁₋₄ alkyl, C₂₋₄ alkenyl, C₇₋₁₁ aralkyl or 5- to 10-membered-heterocyclic-C₁₋₄ alkyl group, each group being optionally substituted by one or more substituents selected from the group consisting of halogen atoms, hydroxyl and formyl groups.

More preferably, R represents a hydrogen atom or a methyl, butyl, formylmethyl, hydroxyethyl, propenyl, benzyl, halobenzyl, especially fluorobenzyl, pyridylmethyl, thienylmethyl or furylmethyl group.

Preferably, R represents a hydrogen atom or a C₁₋₄ alkyl or benzyl group, each group being optionally substituted by one or more halogen atoms or hydroxyl groups.

In a particularly preferred sub-group of compounds, R represents a hydrogen atom or a benzyl, fluorobenzyl or hydroxyethyl group.

Preferably, the parasite is an organism of the genus Neospora or the genus Eimeria.

The present invention also provides the use of a compound of the general formula I as defined above for the manufacture of a medicament for the treatment and/or prophylaxis of a disease caused by infection with a parasite other than an organism of the genus Plasmodium.

The invention is particularly effective when the parasite causing the disease is an organism of the genus Neospora or the genus Eimeria.

According to the present invention, there is also provided a compound of the general formula I as defined above, with the proviso that R is not a hydrogen atom or a methyl, isobutyl, allyl, formylmethyl, pyrid-2-ylmethyl, thenyl or furfuryl group, for use in the treatment and/or prophylaxis of a disease caused by infection with a parasite of the genus Plasmodium.

The present invention also provides the use of a compound of the general formula I as defined in the preceding paragraph for the manufacture of a medicament for the treatment and/or prophylaxis of a disease caused by infection with a parasite of the genus Plasmodium.

Certain compounds of the general formula I are novel and the invention therefore further provides a compound of the general formula I as defined in the preceding paragraph, with the further proviso that R is not a benzyl, cyanoethyl, ethoxycarbonylethyl, ethanoylethyl, phenylsulphinylethyl, phenylsulphonylethyl or phenylsulphonatoethyl group.

It should also be appreciated that the compounds of general formula I are capable of existing as different geometric and optical isoners. The present invention thus includes both the individual isomers and mixtures of such isomers.

The present invention also provides a process for the preparation of a novel compound of the general formula I as defined above which comprises reacting artemisinin with a compound of the general formula RNH₂ where R is as defined above.

The reaction may be conveniently carried out in the presence of a solvent. Suitable solvents include lower alcohols, especially a C₁₋₄ alcohol, such as methanol, and halogenated hydrocarbons, especially a chlorinated hydrocarbon such as dichloromethane. The reaction is preferably carried out under an inert atmosphere, such as a nitrogen atmosphere. Preferably, the reaction is carried out at a temperature of -5 to +5°C, especially about 0°C. It may also be advantageous to add p-toluene sulphonic acid during the course of the reaction to ensure that any intermediate products are converted into the final cyclised lactam of formula I. Also, in some cases, it may be necessary to utilise an amine of formula RNH₂ which has been freshly distilled before the reaction in order to ensure that reaction occurs.

Alternatively, certain compounds of general formula I in which R represents a group -CH₂CH₂R¹, where R¹ represents an electron-withdrawing group, may be prepared in a Michael addition reaction by reacting 11-azaartemisinin with a compound of the general formula CH₂=CH-R¹, where R¹ is as defined above. Preferably, the electron-withdrawing group is a nitro, cyano, formyl, alkanoyl, alkoxycarbonyl, alkylsulphinyl, alkylsulphonyl, alkylsulphonato, arylsulphinyl, arylsulphonyl or arylsulphonato group. It is also preferred that the reaction is carried out in the presence of a base, such as sodium hydroxide, and in the presence of a solvent, such as tetrahydrofuran.

The 11-azaartemisinin starting material may be prepared by reacting artemisinin with ammonia using the first process of the invention described above.

The invention also provides a pharmaceutical composition which comprises a carrier and, as active ingredient, a novel compound of the general formula I as defined above. A process for preparing a pharmaceutical composition which comprises bringing a carrier into association with a novel compound of the general formula I is also provided.

A pharmaceutically acceptable carrier may be any material with which the active ingredient is formulated to facilitate administration. A carrier may be a solid or a liquid, including a material which is normally gaseous but which has been compressed to form a liquid, and any of the carriers normally used in formulating pharmaceutical compositions may be used. Preferably, compositions according to the invention contain 0.5 to 95% by weight of active ingredient.

The compounds of general formula I can be formulated as, for example, tablets, capsules, suppositories or solutions. These formulations can be produced by known methods using conventional solid carriers such as, for example, lactose, starch or talcum or liquid carriers such as, for example, water, fatty oils or liquid paraffins. Other carriers which may be used include materials derived from animal or vegetable proteins, such as the gelatins, dextrins and soy, wheat and psyllium seed proteins; gums such as acacia, guar, agar, and xanthan; polysaccharides; alginates; carboxymethylcelluloses; carrageenans; dextrans; pectins; synthetic polymers such as polyvinylpyrrolidone; polypeptide/protein or polysaccharide complexes such as gelatin-acacia complexes; sugars such as mannitol, dextrose, galactose and trehalose; cyclic sugars such as cyclodextrin; inorganic salts such as sodium phosphate, sodium chloride and aluminium silicates; and amino acids having from 2 to 12 carbon atoms such as a glycine, L-alanine, L-aspartic acid, L-glutamic acid, L-hydroxyproline, L-isoleucine, L-leucine and L-phenylalanine.

Auxiliary components such as tablet disintegrants, solubilisers, preservatives, antioxidants, surfactants, viscosity enhancers, colouring agents, flavouring agents, pH modifiers, sweeteners or taste-masking agents may also be incorporated into the composition. Suitable colouring agents include red, black and yellow iron oxides and FD & C dyes such as FD & C blue No. 2 and FD & C red No. 40 available from Ellis & Everard. Suitable flavouring agents include mint, raspberry, liquorice, orange, lemon, grapefruit, caramel, vanilla, cherry and grape flavours and combinations of these. Suitable pH modifiers include citric acid, tartaric acid, phosphoric acid, hydrochloric acid and maleic acid. Suitable sweetners include aspartame, acesulfame K and thaumatin. Suitable taste-masking agents include sodium bicarbonate, ion-exchange resins, cyclodextrin inclusion compounds, adsorbates or microencapsulated actives.

For treatment of and prophylaxis against coccidiosis and related parasites, for instance, in poultry, especially in chickens, ducks, geese and turkeys, 0.1 to 100 ppm, preferably 0.5 to 100 ppm of the active compound may be mixed into an appropriate, edible material, such as nutritious food. If desired, the amounts applied can be increased, especially if the active compound is well tolerated by the recipient. Accordingly, the active compound can be applied with the drinking water.

For the treatment of a single animal, for instance, for the treatment of coccidiosis in mammals or toxoplasmosis, amounts of 0.5 to 100 mg/kg body weight active compound are preferably administered daily to obtain the desired results. Nevertheless, it may be necessary from time to time to depart from the amounts mentioned above, depending on the body weight of the experimental animal, the method of application, the animal species and its individual reaction to the drug or the kind of formulation or the time or interval in which the drug is applied. In special cases, it may be sufficient to use less than the minimum amount given above, whilst in other cases the maximum dose may have to be exceeded. For a larger dose, it may be advisable to divide the dose into several smaller single doses.

The invention also provides a method for treating a disease caused by infection with a parasite other than an organism of the genus Plasmodium which comprises administering to a host in need of such treatment a therapeutically effective amount of a compound of the general formula I as first defined above. Preferably, the parasite is an organism of the genus Neospora or the genus Eimeria. A method for treating a disease caused by infection with a parasite of the genus Plasmodium is also provided which comprises administering to a host in need of such treatment a therapeutically effective amount of a compound of the general formula I as defined above. Preferably, the host is a mammalian host.

The invention is further illustrated by the following examples.

### Example 1

### Preparation of 11-azaartemisinin (Formula I: R=H)

To a saturated solution of methanolic ammonia (12ml) at room temperature was added artemisinin (1.128g, 4mmol). The solution was stirred for 1.5 hours and concentrated under reduced pressure to give a light yellow solid. The solid was dissolved in dichloromethane (180 ml), and 2,6-di-tert-butyl-4-methylphenol (BHT)(80mg, 0.36 mmol), 15% sulphuric acid (0.8 ml), and silica gel (8.0 g) were added in succession. After stirring overnight at room temperature, the reaction mixture was filtered and the silica gel washed with dichloromethane. The combined organic solution and washes were concentrated under reduced pressure. Column chromatography of the residue on silica gel with acetone: dichloromethane (8:92) gave crystalline solid 11-azaartemisinin (510 mg, 45%) : mp 143-145°C;R_{f} = 0.40 (acetone: CH₂Cl₂, 15:85); [α]²⁵_{D}=-40.9° (c 0.127, CH₂Cl₂); IR 3313, 3223, 2928, 2873, 1668cm⁻¹; CIMS (NH₃) 299 (M + NH₄⁺, 76), 282 (M + 1,100); ¹H NMR δ 0.93-1.01 (2H, m), 0.93 (3H, d, J = 5.5 Hz), 1.07 (3H, d, J=7.2 Hz), 1.30 (3H, s), 1.25-1.42(3H, m), 1.61-1.70 (2H,m), 1.71-1.77 (1H, m), 1.92 (2H, dm, J = 10.2 Hz), 2.34 (1H, m), 3.17 (1H, pent, J= 6.8 Hz), 5.33 (1H, s), 5.93 (1H, bs).

### Example 2

### Preparation of 11-aza-N-(2-fluorobenzyl)artemisinin (Formula I:R=2-F benzyl)

Artemisinin (410mg, 1.44 mmol) was suspended in methanol (4.5 ml) and cooled to 0°C. Freshly distilled 2-fluorobenzylamine (360mg, 2.88 mmol) was added and the reaction mixture was stirred under nitrogen at 0°C for 48 hours. After this time, the solvent was evaporated and the residue quickly dissolved in dichloromethane (67.5ml). Para-toluene sulphonic acid (550mg, 2.88 mmol)) was added and the solution stirred at room temperature overnight, extracted with 5% NaHCO₃ solution and water, dried over magnesium sulphate and evaporated. Flash chromatography (10% ethyl acetate/hexane) gave 11-aza-N-(2-fluorobenzyl)artemisinin (210 mg, 37%) as a yellow viscous liquid. Recrystallisation from light petroleum ether gave white needles:
M.p. 70.5-71°C; [α]_{D}^{20.5} + 5.55° (c. 0.0182 in CHC1₃); νₘₐₓ (Neat): 2928, 1660 (ν_{c=0,}, amide), 1586, 1490, 1446, 1378, 1330, 1270, 1228, 1204, 1136, 1098, 1072, 1028, 1002, 952, 918, 88, 836, 756, 700 cm⁻¹; ¹H NMR (300 MHz, CDCl₃): δ_{H} (ppm) = 0.83-1.08 (m, 1 H), 0.97(d, J_{6-Me,6}=5.8 Hz, 3H, 6-CH₃), 1.02(s, 3H, 3-CH₃), 1.18 (d, *J*_{*9-Me,9*}*=7.4 Hz, 3H,* 9-CH₃), *1.25-1.51 (m, 3H), 1. 65-1. 76* (m, 3H), 1.80 (td, J=12.9 Hz, J=3.6 Hz, 1H), 1.91-2.04 (m, 2H), 2.32-2.43 (m, 1H), 3.40 (dq, J_{9,9-Me}=7.4 Hz, J_{9,8a}=4.9 Hz, 1H, H-9), 4.67 (d, J=15.2 Hz, 1H, H-1'β), 4.89 (d, *J*=15.2 Hz, 1H, H-1'α), 5.24 (s, 1H, H-12), 6.96-7-38 (m, 4H, phenyl-H); MS (FAB: m/z(%)=390 [M⁺ + 1] (56), 357 [M⁺- O₂] (4), 307 (24), 289 (12), 165 (8), 154 (100), 136 (68); Anal. Calc. for C₂₂H₂₈FNO₄: C, 67.85; H, 7.25; N, 3.59; Found: C, 67.81; H, 7.55; N, 3.48.

### Example 3

### Preparation of 11-aza-N-(ethoxycarbonylethyl)-artemisinin (Formula I: R= -CH₂CH₂COOC₂H₅)

To a solution of 11-azaartemisinin produced as described in Example 1 (27mg, 0.01 mmol) in anhydrous tetrahydrofuran (2 ml) were added at room temperature a trace of powdered sodium hydroxide and freshly distilled ethyl acrylate (31 µl, 0.03 mmol). After stirring for one hour, another trace of sodium hydroxide was added and stirring continued for one additional hour. The reaction was then quenched by adding dilute aq. sodium sulphite solution (5 ml). The water layer was extracted with dichloromethane (3 x 10 ml). The combined organic layers were successively washed with saturated aqueous sodium hydrogen carbonate, brine, water, and dried over sodium sulphate. Evaporation of the solvent gave a crude oily product which was purified by silica gel chromatography (ethyl acetate/hexane, 4:1). This gave N-ethoxycarbonyl- ethyl-11-azaartemisinin as a clear oil (31.6 mg, 86%): CIMS (NH₃) 399 (M+NH₄⁺, 100), 382 (M+1,90); ¹H NMR (CDC1₃, 300 MHz) δ 0.92-0.98 (2H, m) 0.99 (3H, d, J = 5.97 Hz), 1.13 (3H, d, J = 7.16 Hz), 1.25 (3H, t, J = 7.06 Hz), 1.37 (3H, s), 1.26-1.43 (3H, m), 1.63-1.78 (3H, m), 2.01 (2H, dm, J = 10.1 Hz), 2.41 (1H, m), 2.50-2.61 (1H, m), 2.75-2.85 (1H, m), 3.27 (1H, p, J = 6.95 Hz), 3.61-3.70 (1H, m), 3.79-3.88 (1H, m), 4.12 (2H, q, J = 7.13 Hz), 5.33 (1H, s); ¹³C NMR (CDCl₃, 75.4 MHz) δ 13.2, 14.5, 20.1, 23.0, 25.4, 25.8, 33.0, 33.5, 34.0, 37.0, 37.9, 38.8, 45.9, 51.8, 60.8, 79.2, 80.5, 105.2, 172.2, 172.9.

### Examples 4 to 14

By processes similar to those described in Examples 1, 2 and 3 above, further compounds according to the invention were prepared as detailed in Table I below. In this table, the compounds are identified by reference to formula I.

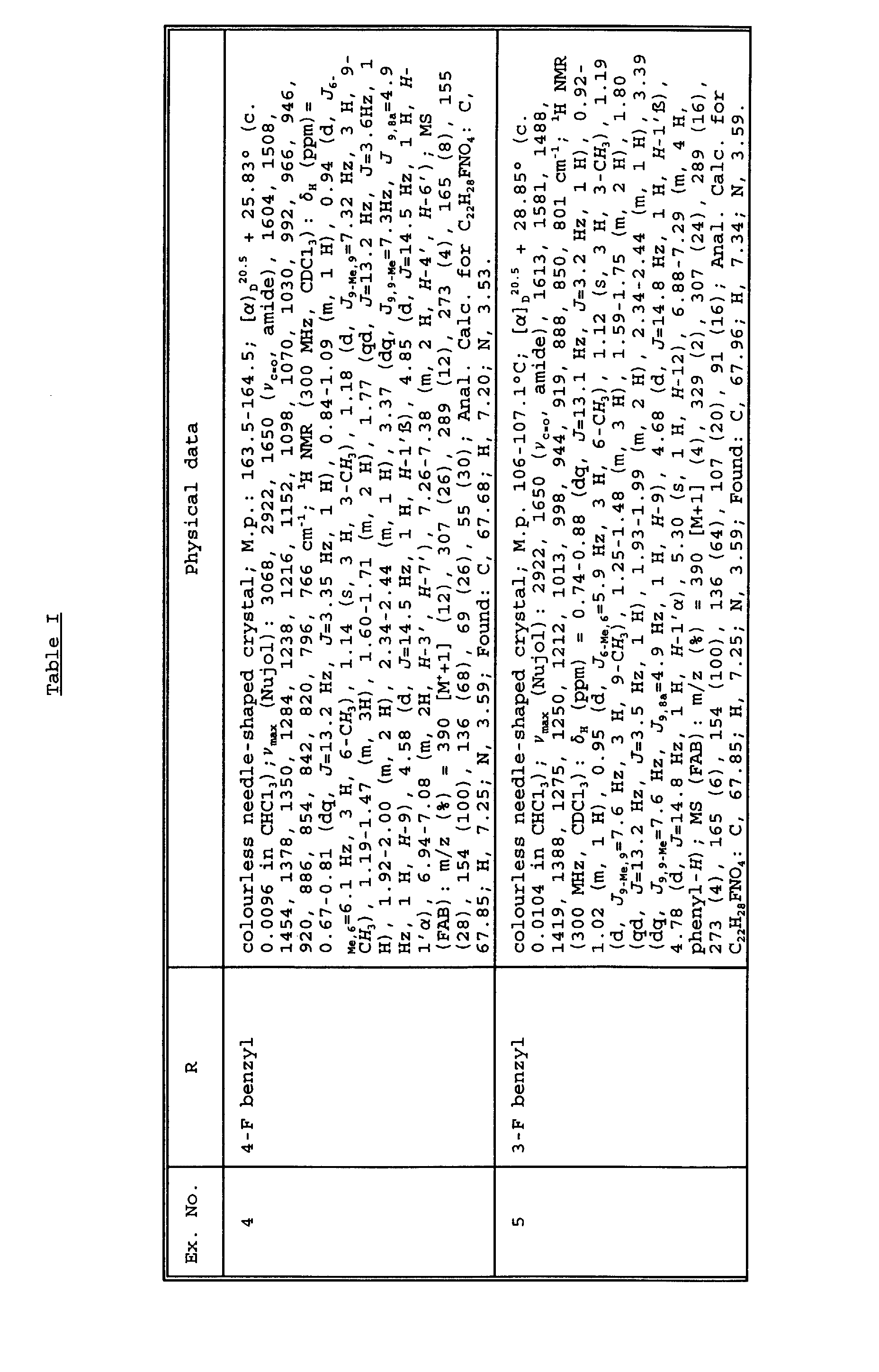

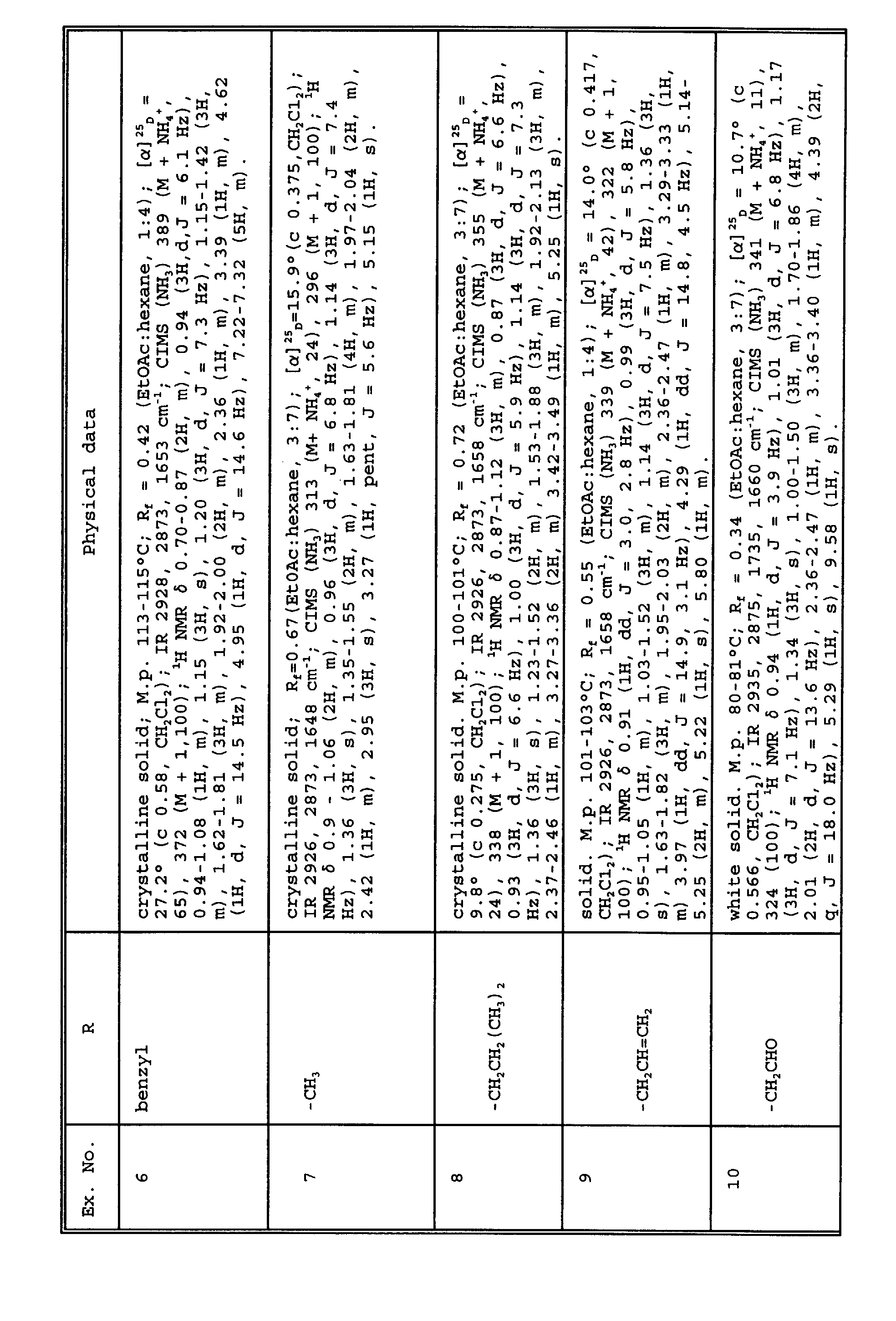

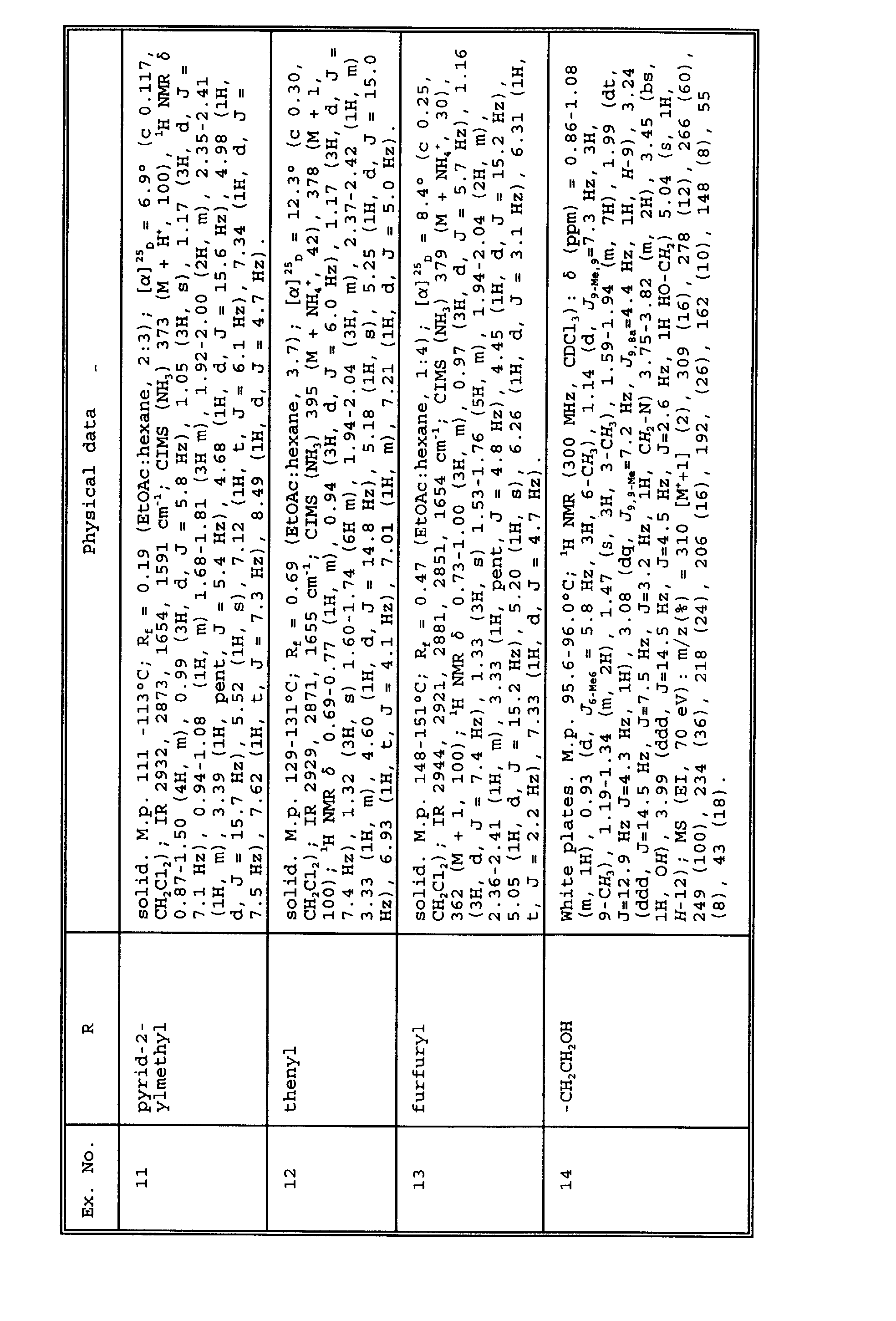

### Example 15

The parasitical activity of compounds of the invention was investigated by means of the following test. Abbreviations used in the example:
RPMI = growth medium for cell cultures.

### (a) InVitro Activity against Plasmodium Falciparum

Two parasite strains - W2 resistant to chloroquine, and D6 sensitive to chloroquine but resistant to mefloquine were used. In Table I below, the best compounds should show no cross resistance between the two strains.

The assay relies on incorporation of radiolabelled hypoxanthine by the parasite and inhibition of incorporation is attributed to activity of known or candidate antimalarial drugs. For each assay, proven antimalarials such as chloroquine, mefloquine, quinine, artemisinin and pyrimethamine were used as controls. The incubation period was 66 hours, and the starting parasitemia was 0.2% with 1% hematocrit. The medium was an RPMI-1640 culture with no folate or p-aminobenzoic acid. Albumax rather than 10% normal heat inactivated human plasma was used as, with Albumax, less protein binding is observed, and compounds elicit slightly higher activities in this model. If a compound was submitted with no prior knowledge of activity, it was dissolved directly in dimethyl sulphoxide (DMSO), and diluted 400 fold with complete culture medium. The unknown compound was started at a maximum concentration of 50,000 ng ml⁻¹ and sequentially diluted 2-fold for 11 times to give a concentration range of 1048 fold. These dilutions were performed automatically by a Biomek 1000 Liquid Handling System in 96-well microtiter plates. The diluted drugs were then transferred to test plates, 200 µL of parasitized erythrocytes were added, and incubated at 37°C in a controlled environment of 5% CO₂, 5% O₂ and 90% N₂. After 42 hours, 25 µL of ³H-hypoxanthine was added, and the plates incubated for an additional 24 hours. After 66 hours, the plates were frozen at -70°C to lyse the red cells, and then thawed and harvested onto glass fiber filter mats in a 96-well harvester. The filter mats were then counted in a scintillation counter. For each drug, the concentration response profile was determined and 50% and 90% inhibitory concentrations (IC₅₀, IC₉₀ and IC₁₀) were determined by a non-linear logistic dose response analysis program.

A prescreen format can be used wherein a 3-dilution assay may be used to determine activity at high medium or low concentrations. The concentrations were selected as 50,000, 500 and 50 ng ml⁻¹. These were performed in duplicate on a 96-well format plate with 14 test compounds and one known (standard) compound per plate. The system was automated with a Biomek diluter for mixing and diluting the drugs, and adding drugs and parasites to a test plate.

In the prescreen format, if the ANALYSIS FIELD (AF) has a "<", then the compound was "very active" and the IC values are most likely to be below the last dilution value (in nanograms/ml), which is listed next to AF. In most cases, these compounds were run again at lower starting concentration to determine the true IC value. If the AF has a ">", then the IC value is greater than the prescreen dilution value; thus "AF>250" means that the IC value is greater than 250 ng ml⁻¹ and no further screening is carried out. In such cases, values of 0.00 are entered for IC values.

The results are set out in Table II below:-

**Table II**

| Example No. | In vitro activity: IC₅₀; IC₉₀; (IC₁₀) ng/ml | |
|---|---|---|
| | W2 strain (chloroquine resistant) | D6 strain (chloroquine sensitive |
| 1 | 1.73; 2.24 | 2.60; 3.60 |
| 2 | 0.775; 0.975 | 0.64; 0.99 |
| 4 | 42.11; 51.63 0.53; 0.92; (0.31) | 35.55; 49.77 1.51; 2.02; (1.13) |
| 5 | 0.58; 0.95 | 0.79; 1.26 |
| 6 | 3.795; 4.68 | 3.62; 6.68 |
| 14 | 31.19; 37.01; (26.29) | 162.19; 446.47; (58.92) |

## Claims

1. A compound of the general formula I 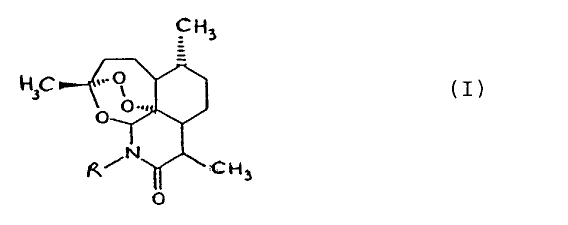 in which
R represents a hydrogen atom or an optionally substituted alkyl, alkenyl, alkynyl, aralkyl or heterocyclylalkyl group;
for use in the treatment and/or prophylaxis of a disease caused by infection with a parasite other than an organism of the genus Plasmodium

2. A compound according to claim 1 in which R represents a hydrogen atom or a C₁₋₆ alkyl, C₂₋₆ alkenyl, C₂₋₆alkynyl, C₇₋₁₆ aralkyl or 5- to 10-membered-heterocyclic-C₁₋₆ alkyl group, each group being optionally substituted by one or more substituents selected from the group consisting of halogen atoms, cyano, nitro, C₁₋₄ haloalkyl, formyl, C₁₋₆ alkoxycarbonyl, C₁₋₆ alkanoyl, phenylsulphinyl, phenylsulphonyl and phenylsulphonato groups.

3. A compound according to claim 1 or claim 2 in which R represents a hydrogen atom or a C₁₋₄ alkyl or benzyl group, each group being optionally substituted by one or more halogen atoms or hydroxyl groups.

4. A compound according to any one of the preceding claims in which R represents a hydrogen atom or a benzyl, fluorobenzyl or hydroxyethyl group.

5. A compound according to any one of the preceding claims in which the parasite is an organism of the genus Neospora or the genus Eimeria.

6. Use of a compound of the general formula I as defined in any one of claims 1 to 4 for the manufacture of a medicament for the treatment and/or prophylaxis of a disease caused by infection with a parasite other than an organism of the genus Plasmodium.

7. Use according to claim 6 in which the parasite is an organism of the genus Neospora or the genus Eimeria.

8. A compound of the general formula I as defined in any one of claims 1 to 4, with the proviso that R is not a hydrogen atom or a methyl, isobutyl, allyl, formylmethyl, pyrid-2-ylmethyl, thenyl or furfuryl group, for use in the treatment and/or prophylaxis of a disease caused by infection with a parasite of the genus Plasmodium.

9. Use of a compound of the general formula I as defined in claim 8 for the manufacture of a medicament for the treatment and/or prophylaxis of a disease caused by infection with a parasite of the genus Plasmodium.

10. A compound of the general formula I as defined in claim 8, with the further proviso that R is not a benzyl, cyanoethyl, ethoxycarbonylethyl, ethanoylethyl, phenylsulphinylethyl, phenylsulphonylethyl or phenylsulphonatoethyl group.

11. A process for the preparation of a compound of the general formula I according to claim 10 which comprises reacting artemisinin with a compound of the general formula RNH₂, where R is as defined in claim 10.

12. A process for the preparation of a compound of the general formula I according to claim 10 in which R represents a group -CH₂CH₂R¹ where R¹ represents an electron-withdrawing group which comprises reacting 11-azaartemisinin with a compound of the general formula CH₂=CH-R¹, where R¹ is as defined above.

13. A pharmaceutical composition which comprises a carrier and, as active ingredient, a compound of the general formula I according to Claim 10.

14. A method for treating a disease caused by infection with a parasite other than an organism of the genus Plasmodium which comprises administering to a host in need of such treatment a therapeutically effective amount of a compound of the general formula I as defined in claim 1.

15. A method for treating a disease caused by infection with a parasite of the genus Plasmodium which comprises administering to a host in need of such treatment a therapeutically effective amount of a compound of the general formula I as defined in claim 8.
